# EUROPEAN PATENT SPECIFICATION

(11) **EP 4 152 334 B1**
(45) Date of publication and mention of the grant of the patent: **23.07.2025**
(21) Application number: 22817861.2
(22) Date of filing: 06.06.2022
(51) Int. Cl.: G16B 30/20, G16B 50/00, G06F 9/50, G16B 20/20, G16B 40/20

(54) **GENE SEQUENCING ANALYSIS METHOD AND APPARATUS, AND STORAGE MEDIUM AND COMPUTER DEVICE**
VERFAHREN UND VORRICHTUNG ZUR GENSEQUENZIERUNGSANALYSE SOWIE SPEICHERMEDIUM UND COMPUTERVORRICHTUNG
PROCÉDÉ ET APPAREIL D'ANALYSE DE SÉQUENÇAGE DE GÈNES, ET SUPPORT DE STOCKAGE ET DISPOSITIF INFORMATIQUE

(30) Priority: 23.06.2021 CN 202110698855
(43) Date of publication of application: 22.03.2023
(73) Proprietor: BGI Genomics Co., Limited, Guangdong 518083 (CN)
(72) Inventor: YANG, Jiaobo, Shenzhen Guangdong 518083 (CN); JIN, Xiangqian, Shenzhen Guangdong 518083 (CN); HE, Zengquan, Shenzhen Guangdong 518083 (CN); ZHANG, Youjin, Shenzhen Guangdong 518083 (CN)
(74) Representative: Dehns Germany Partnerschaft mbB
(86) International application number: PCT/CN2022/097102
(87) International publication number: WO 2022/267867

(56) References cited:
- CN-A- 109 801 677
- CN-A- 111 524 552
- CN-A- 112 259 168
- CN-A- 112 410 408
- CN-A- 113 299 344
- US-A1- 2020 131 509
- MARTIN S. LINDNER ET AL: "HiLive - Real-Time Mapping of Illumina Reads while Sequencing", BIOINFORMATICS, vol. 33, no. 6, 19 December 2016 (2016-12-19), GB, pages 917 - 919, XP055600765, ISSN: 1367-4803, DOI: 10.1093/bioinformatics/btw659
- MARTIN S LINDNER ET AL: "Supplementary Material: HiLive -Real-Time Mapping of Illumina Reads while Sequencing", BIOINFORMATICS, 19 December 2016 (2016-12-19), pages 1 - 7, XP093100289, Retrieved from the Internet <URL:https://academic.oup.com/bioinformatics/article/33/6/917/2567469> [retrieved on 20231110], DOI: https://doi.org/10.1093/bioinformatics/btw659
- TOBIAS P. LOKA ET AL: "Reliable variant calling during runtime of Illumina sequencing", SCIENTIFIC REPORTS, vol. 9, no. 1, 11 November 2019 (2019-11-11), XP055750387, DOI: 10.1038/s41598-019-52991-z
- KAREL B\V{R}INDA ET AL: "Dynamic read mapping and online consensus calling for better variant detection", ARXIV.ORG, CORNELL UNIVERSITY LIBRARY, 201 OLIN LIBRARY CORNELL UNIVERSITY ITHACA, NY 14853, 30 May 2016 (2016-05-30), XP080704370

## Description

### FIELD

The present disclosure relates to the technical field of biological information, and particularly, to a gene sequencing analysis method and apparatus, a storage medium, and computer device.

### BACKGROUND

DNA sequencing technology has been playing a vital role in driving the development of molecular biology since its emergence. This technical field has undergone tremendous changes from early manual sequencing developed by Frederick Sanger and the first-generation automated sequencers based on the Sanger's sequencing, to the current new-generation sequencing platforms.

The sequencing process of the new generation sequencing platform mainly includes the following steps: during each cycle of sequencing, adding four nucleotides labeled with different fluorophores and DNA polymerases simultaneously into flow-through channels, to extend the DNA chain in accordance with the principle of complementary pairing; acquiring fluorescence images, with base-specific fluorescence labeling revealing which nucleotides are newly added to the chain in this cycle, so as obtain the DNA sequences at this position in the template; and further performing the next cycle of reaction. Such a process is repeated multiple times to obtain multiple DNA sequences. For example, a DNA sequence including 50 bases can be obtained by performing 50 cycles of sequencing. The single sequence obtained by this method is very short, and thus it is also referred to as a short sequence.

In this regard, for the new-generation sequencing platforms, it is necessary to complete the biochemical and imaging operations before base recognition, and the recognition results shall be converted into other forms for storage. Thereafter, the stored files are transmitted to a primary storage for performing data quality control, checking sample information, and sequencing information, and data is archived using the secondary storage. Such a sequencing is time-consuming and has a low transmission efficiency, resulting in a relatively slow analysis process. Martin S. Lindner et al., present HiLive as a novel real time read mapper that implements a k-mer based alignment strategy. Tobias P. Loka et al., present a novel real-time read mapping algorithm with fast variant calling to obtain reliable variant calls still during the sequencing process. Binda et al., propose a dynamic read mapping approach that significantly improves read alignment accuracy.

### SUMMARY

The present disclosure aims to solve at least one of the above-mentioned technical drawbacks, in particular the technical drawbacks of the related art such as time-consuming sequencing and low transmission efficiency, which further leads to the relatively slow analysis process.

In a first aspect, the present disclosure provides a gene sequencing analysis method. The gene sequencing analysis method includes the following steps of: acquiring slice data transmitted from a sequencing platform in real time, the slice data being a set of short sequences read after performing at least one cycle of sequencing on a sequencing library; inputting the slice data into a memory; and performing, by calling an encapsulation program pre-set in the memory, data processing and detection analysis on the slice data, to obtain a corresponding analysis result, wherein the encapsulation program is a custom program; wherein the encapsulation program comprises a data quality control program, a data processing program, and a detection analysis program, and wherein said performing, by calling the encapsulation program pre-set in the memory, the data processing and detection analysis on the slice data, to obtain the corresponding analysis result comprises: performing, by calling the data quality control program pre-set in the memory, quality control processing on the slice data, to obtain a quality control result; performing, by calling the data processing program pre-set in the memory, data processing on the quality control result, to obtain a processing result; and performing, by calling the detection analysis program pre-set in the memory, detection analysis on the processing result, to obtain a final analysis result; wherein acquiring the slice data includes: determining a slice size; slicing, based on the slice size, all unsliced short sequences currently transmitted from the sequencing platform and read after a single cycle of sequencing, a set of the sliced short sequences being used as the slice data; wherein determining the slice size includes: acquiring a current central processing unit (CPU) idleness state; and adjusting the slice size based on the current CPU idleness state; wherein when the memory is not idle, the slice size is adjusted to be able to slice a set of short sequences that are read after multiple cycles of sequencing; and when the memory is idle, the slice size is adjusted to be able to slice a set of short sequences that are read after one cycle of sequencing; wherein biochemical and imaging parts in sequencing process of each cycle are transmitted to the memory in real time for base recognition, after corresponding short sequences are obtained, the slice size is determined first, and the short sequences satisfying the slice size, as a combination, is output as the slice data, and the slice data is stored in the memory; wherein every set of multiple cycles of sequencing is taken as one piece of slice data, divided into a first slice and subsequent slices, and the slice size can be adaptively adjusted according to the analysis speed; where the first slice contains multiple sequencing cycles; the subsequent slices are automatically controlled: if the machine is relatively idle, the transmission is performed once after each cycle, the subsequent slices only contain one sequencing cycle; and if the machine is relatively busy, the transmission can be selected to perform after a suitable sequencing cycle number of the subsequent slices.

According to an embodiment of the present disclosure, the above-mentioned gene sequencing analysis method may further comprise at least one of the following additional technical features.

According to an embodiment of the present disclosure, the encapsulation program includes a data quality control program, a data processing program, and a detection analysis program. The step of performing, by calling the encapsulation program pre-set in the memory, the data processing and detection analysis on the slice data, to obtain the corresponding analysis result includes: performing, by calling the data quality control program pre-set in the memory, quality control processing on the slice data, to obtain a quality control result; performing, by calling the data processing program pre-set in the memory, data processing on the quality control result, to obtain a processing result; and performing, by calling the detection analysis program pre-set in the memory, detection analysis on the processing result, to obtain a final analysis result.

According to an embodiment of the present disclosure, the step of performing, by calling the data quality control program pre-set in the memory, the quality control processing on the slice data includes: by calling the data quality control program pre-set in the memory, calculating a position error rate of short sequences in the slice data, statistically analyzing a base distribution in the slice data, and/or removing low-quality bases from the slice data, wherein the low-quality bases are bases with a base quality below a quality threshold.

According to an embodiment of the present disclosure, the step of performing, by calling the data processing program pre-set in the memory, the data processing on the quality control result includes: performing, by calling the data processing program pre-set in the memory, at least one of alignment, ordering, deduplication, and re-alignment on a short sequence in the quality control result.

According to an embodiment of the present disclosure, the step of performing, by calling the data processing program pre-set in the memory, at least one of alignment, ordering, deduplication, and re-alignment on the short sequence in the quality control result includes: performing, by calling the data processing program pre-set in the memory, an alignment between the short sequence in the quality control result and a reference gene, to determine a position of the short sequence on the reference gene; determining a position tag of the short sequence based on the position of the short sequence on the reference gene; determining, among pre-set threads in the memory, a target thread that is currently in an idle state; and performing, by calling the target thread and with reference to the position tag of the short sequence, at least one of ordering, deduplication, and re-alignment of the short sequence.

According to an embodiment of the present disclosure, the step of performing, by calling the detection analysis program pre-set in the memory, the detection analysis on the processing result includes: performing, by calling the detection analysis program pre-set in the memory, mutation detection and interpretation on the processing result.

In a second aspect, the present disclosure further provides a gene sequencing analysis apparatus. The gene sequencing analysis apparatus includes: a data acquisition module configured to acquire slice data transmitted from a sequencing platform in real time, the slice data being a set of short sequences read after performing at least one cycle of sequencing on a sequencing library; a slice transmission module configured to input the slice data into a memory; and a processing analysis module configured to perform, by calling an encapsulation program pre-set in the memory, data processing and detection analysis on the slice data, to obtain a corresponding analysis result, wherein the encapsulation program is a custom program; wherein the encapsulation program comprises a data quality control program, a data processing program, and a detection analysis program, and wherein the processing analysis module is further configured to: perform, by calling the data quality control program pre-set in the memory, quality control processing on the slice data, to obtain a quality control result; perform, by calling the data processing program pre-set in the memory, data processing on the quality control result, to obtain a processing result; and perform, by calling the detection analysis program pre-set in the memory, detection analysis on the processing result, to obtain a final analysis result; wherein the data acquisition module is further configured to: determine a slice size; slice, based on the slice size, all unsliced short sequences currently transmitted from the sequencing platform and read after a single cycle of sequencing, a set of the sliced short sequences being used as the slice data; wherein for determining the slice size, the data acquisition module is further configured to: acquire a current central processing unit (CPU) idleness state; and adjust the slice size based on the current CPU idleness state; wherein when the memory is not idle, the slice size is adjusted to be able to slice a set of short sequences that are read after multiple cycles of sequencing; and when the memory is idle, the slice size is adjusted to be able to slice a set of short sequences that are read after one cycle of sequencing; wherein biochemical and imaging parts in sequencing process of each cycle are transmitted to the memory in real time for base recognition, after corresponding short sequences are obtained, the slice size is determined first, and the short sequences satisfying the slice size, as a combination, is output as the slice data, and the slice data is stored in the memory; wherein every set of multiple cycles of sequencing is taken as one piece of slice data, divided into a first slice and subsequent slices, and the slice size can be adaptively adjusted according to the analysis speed; where the first slice contains multiple sequencing cycles; the subsequent slices are automatically controlled: if the machine is relatively idle, the transmission is performed once after each cycle, the subsequent slices only contain one sequencing cycle; and if the machine is relatively busy, the transmission can be selected to perform after a suitable sequencing cycle number of the subsequent slices.

In a third aspect, the present disclosure also provides a storage medium having computer readable instructions stored thereon. The computer readable instructions, when being executed by one or more processors, cause the one or more processors to perform steps of the gene sequencing analysis method as described in any of the above embodiments.

In a fourth aspect, the present disclosure also provides a computer device having computer readable instructions stored thereon. The computer readable instructions, when being executed by one or more processors, cause the one or more processors to perform steps of the gene sequencing analysis method as described in any of the above embodiments.

It can be seen from the above technical solutions that the embodiments of the present disclosure have the following advantages.

In the gene sequencing analysis method and apparatus, the storage medium, and the computer device according to the present disclosure, short sequences transmitted from a sequencing platform in real time are first sliced to perform corresponding slicing processing, and then slice data is input into a memory. Thus, a pre-loaded encapsulation program in the memory can be called to perform bioinformatic analysis on the slice data, thereby obtaining a corresponding analysis result. Such a process can acquire the slice data of the sequencing of the sequencing platform in real time and perform sequencing and analysis on the slice data, rather than performing the processing analysis only after the sequencing platform has finished the sequencing and the complete sequencing results are transmitted to the corresponding platform. Therefore, the present disclosure enables the whole of sequencing and analysis to be accelerated. In addition, the data for analysis is the slice data, which can be transmitted faster and takes less time compared with the whole sequencing result.

In addition, the analysis process according to the present disclosure is performed by means of a pre-set encapsulation program in memory. Thus, the sequencing data is unnecessarily transmitted to another platform for processing, which effectively reduces I/O congestion and memory consumption, thereby further improving the operating efficiency of a machine and prolonging the service life of the machine.

### DESCRIPTION OF THE DRAWINGS

To clearly explain the technical solutions in the embodiments of the present disclosure or the related art, the accompanying drawings required for describing the embodiments or the related art are briefly introduced below. The accompanying drawings in the following description merely illustrate some embodiments of the present disclosure. Those skilled in the art can derive other drawings from these accompanying drawings without paying creative efforts.
FIG. 1 is a schematic flowchart of a gene sequencing analysis method according to an embodiment of the present disclosure;
FIG. 2 is a schematic diagram of a flow analysis process according to an embodiment of the present disclosure;
FIG. 3 is a schematic diagram of a gene sequencing analysis process according to an embodiment of the present disclosure;
FIG. 4 is a schematic diagram illustrating a sequencing process in correlation with time in a gene sequencing analysis process according to an embodiment of the present disclosure;
FIG. 5 is a schematic structural diagram of a gene sequencing analysis apparatus according to an embodiment of the present disclosure; and
FIG. 6 is a schematic diagram of an internal structure of a computer device according to an embodiment of the present disclosure.

### DETAILED DESCRIPTION

Technical solutions in the embodiments of the present disclosure are clearly described in detail below with reference to the accompanying drawings of the embodiments of the present disclosure. The embodiments described below are merely part of rather than all the embodiments of the present disclosure. Other embodiments, which are obtained by those skilled in the art based on the embodiments in the present disclosure without paying creative efforts, shall fall within the protection scope of the present disclosure.

In short, sequencing is the conversion from DNA chemical signals into computer-processable digital signals. From early manual sequencing developed by Frederick Sanger and the first-generation automated sequencers based on the Sanger sequencing, to the current new-generation sequencing platforms, the field of sequencing has undergone tremendous changes. The changes and breakthrough in sequencing technology play a great promotion role in the genomics research, disease medical research, drug research, breeding, and other fields.

At present, the sequencing process of the new generation sequencing platform mainly includes the following steps: during each cycle of sequencing, adding four nucleotides labeled with different fluorophores and DNA polymerases simultaneously into flow-through channels, to extend the DNA chain in accordance with the principle of complementary pairing; acquiring fluorescence images, with base-specific fluorescence labeling revealing which nucleotides are newly added to the chain in this cycle, so as obtain the DNA sequences at this position in the template; and further performing the next cycle of reaction. Such a process is repeated multiple times to obtain multiple DNA sequences. For example, a DNA sequence including 50 bases can be obtained by performing 50 cycles of sequencing. The single sequence obtained by this method is very short, and thus it is also referred to as a short sequence.

In view of the above, for the new-generation sequencing platforms, it is necessary to complete the biochemical and imaging operations before base recognition, and the recognition results shall be converted into other forms for storage. Thereafter, the stored files are transmitted to a primary storage for performing data quality control, checking sample information, and sequencing information, and data is archived using the secondary storage. Such a sequencing is time-consuming and has a low transmission efficiency, resulting in a relatively slow analysis process.

Therefore, the present disclosure aims to solve the technical problems of the slow analysis process caused by long time consumption and low transmission efficiency of the sequencing in the related art. The present disclosure provides the following technical solutions.

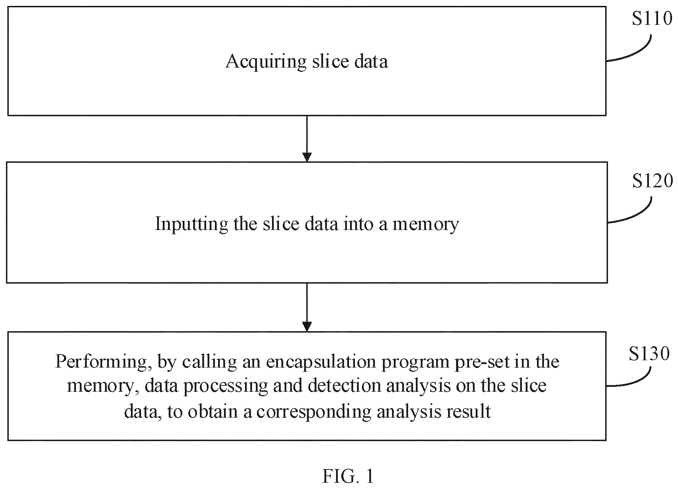

FIG. 1 is a schematic flowchart of a gene sequencing analysis method according to an embodiment of the present disclosure. As illustrated in FIG. 1, the present disclosure provides a gene sequencing analysis method, which specifically includes the following steps.

S110: slice data is acquired.

In this step, before performing bioinformatic analysis, the slice data transmitted from the sequencing platform is required to be acquired, in order to perform the corresponding bioinformatic analysis based on the slice data.

It can be understood that, in the present disclosure, since the analysis efficiency of the subsequent platforms and the occupation situation of the memory, etc. are taken into consideration, the real-time incoming data from the sequencing platform is required to be sliced, and the slice size can be adaptively adjusted based on the idle degree of the server. Therefore, in the present disclosure, the acquired slice data is a set of short sequences read after performing at least one cycle of sequencing on a sequencing library and transmitted from the sequencing platform in real time.

It should be noted that, the adaptive adjustment of the slice size based on the idle degree of the server may mainly include the following situations. If the server is not idle, the slice size can be adjusted to be able to slice short sequences that can be read after one cycle of sequencing. If the server is idle, the slice size can be adjusted to be able to slice short sequences that can be read after multiple cycles of sequencing. Further, the first slicing may occur after multiple cycles of sequencing have been performed. For example, after 10 cycles of sequencing, one slice is made by slicing.

As an example, in the present disclosure, a next-generation sequencing platform may be employed to acquire the slice data. Next-Generation Sequencing (NGS) and Sanger sequencing are the same in that a computer is used to detect the fluorescent signals generated at the time of binding the fluorophore-labelled dNTPs to a DNA template with catalyst of DNA polymerase in each cycle of sequencing. However, unlike Sanger sequencing in which a single fragment is detected per unit time, NGS can detect signals from thousands of channels simultaneously, thereby greatly improving efficiency.

It can be understood that the next-generation sequencing platforms discussed herein include, but are not limited to, Illumina (Solexa) sequencing, Roche 454 sequencing, Ion torrent: Proton/PGM sequencing, and SOLiD sequencing.

As an example, the following explains how the next-generation sequencing platform obtains the slice data. Illustratively, the next-generation sequencing platforms in the present disclosure require a construction of a sequencing library before sequencing. The sequencing library refers to a DNA mixture of DNA fragments having specific DNA adapter sequences connected at both ends thereof. For example, a mixture of DNA fragments is obtained by disrupting genomic DNA with ultrasonics, end-filling the disrupted DNA fragments with enzymes, and ligating the adapter sequences by means of ligases, and such a mixture of DNA fragments is referred to as a "library".

Before the sequencing library is sequenced using the next-generation sequencing platform, PCR amplification may be performed on the sequencing library, in order to amplify the small amount of DNA fragments in the solution to be sequenced by several times or even dozens of times. In this way, a distribution density of the DNA fragments to be sequenced in the solution can be increased, enabling the small amount of DNA fragments to be acquired during sampling.

During the sequencing, a neutral solution may be added, and sequencing dNTP primers may be added to the neutral solution. Since 3'-end of dNTP is blocked by an azide group, the template chain can only be extended by one base during one cycle of sequencing. After one cycle of sequencing is completed, the azide group and fluorophore are cleaved by adding a specific chemical reagent, to expose the hydroxyl group at the 3'-end of dNTP, and thus the base sequence can be read.

In the present disclosure, the biochemical and imaging parts of each cycle of sequencing in the sequencing process are transmitted to the memory in real time for base calling (Basecall) to obtain the corresponding short sequence, and the slice data, when satisfying the slice size, is output and stored in the memory.

For example, a fastq file is output after sequencing with the existing next-generation sequencing platform. The fastq file is a sequence file containing a quality value, where q indicates quality, for storing the original sequencing data, with an extension name of fastq or fq. The following is a common format of sequence in fastq files:
@DJB775P1:248:D0MDGACXX:7:1202:12362:49613
TGCTTACTCTGCGTTGATACCACTGCTTAGATCGGAAGAGCACACGTCTGAA +
JJJJJIIJJJJJJHIHHHGHFFFFFFCEEEEEDBD?DDDDDDBDDDABDDCA
@DJB775P1:248:D0MDGACXX:7:1202:12782:49716
CTCTGCGTTGATACCACTGCTTACTCTGCGTTGATACCACTGCTTAGATCGG +
IIIIIIIIIIIIIIIHHHHHHFFFFFFEECCCCBCECCCCCCCCCCCCCCCC

In view of the above format of sequence, every four rows in the fastq file are regarded as an independent unit, i.e., referring to as a short sequence. The first row begins with "@", followed by DJB775P1 indicating the name of the short sequence, and the character string of the first row without space therebetween is converted according to the state information during sequencing and is a unique identifier of the short sequence. The second row indicates a sequence of sequencing read, i.e., the DNA sequence of real interest. The sequence is composed of five letters of A, C, G, T, and N, where N represents a bases that cannot be identified during the sequencing. The third row begins with "+", and may repeat the information of the first row in the previous version of the FASTQ file. Currently, no information is added to the third row for saving storage space. The fourth row indicates the quality value of the sequencing read, which is as important as the base information of the second row, and the quality value of the sequencing read represents the reliability of each sequenced base, expressed by the ASCII code.

In the present disclosure, in order to improve the analysis efficiency, the sequencing and analysis processes are subjected to slicing, and a stream structure is used for sequential processing to simultaneously perform sequencing, encoding, transmission, and analysis. The result obtained in the biochemistry and imaging parts of each cycle of sequencing in the sequencing process is first transmitted to the memory for image recognition processing of the basecall (base calling) process, to obtain a plurality of short sequences. Thereafter, the slice size is determined based on the idle degree of the server. That is, the bases in the above-mentioned second row are sliced based on the idle degree of the server. If the server is idle, the bases are sliced as one piece of slice data. If the server is not idle, a single base is sliced as one piece of slice data. The slice data obtained after the slicing is saved in the memory.

When reading the slice data in the memory for analysis, the adapter can be first removed. The adapter is located at the beginning of the first slice, generally 6 to 8 bases, and the adapter data can be used to distinguish different samples, which is convenient for later output. Then, the remaining slice data is aligned on the human genome. Each base of each new piece of slice data enables a range of reads to be located on the human genome to continuously narrowed, and thus the alignment position is more accurate.

In addition, in the present disclosure, during the sequencing and analysis, the used platform may be a machine integrating sequencing and analysis, or it may be a machine connecting an existing sequencer and an analyzer through a network connection for data transmission and operation analysis, which is not limited herein.

S120: the slice data is input into memory.

In this step, after the slice data is acquired in step S110, the slice data can be input into a memory of the integration machine or a memory of an analyzer in connection with a sequencer. Thus, the data processing and detection analysis can be performed on the slice data by means of an encapsulation program in the memory.

It can be understood that, after the sequencing library is sequenced with an existing sequencer, the obtained sequencing result is generally output as a fastq file and stored in a hard disk. When performing alignment analysis, the fastq file is read from the hard disk, and aligned to the human genome (reference. fa). In contrast, in the present disclosure, the acquired slice data is directly transmitted to the memory for performing the alignment processing, without being output and transmitted to a hard disk, thereby reducing the processes of writing to and reading from the hard disk and reducing I/O consumption.

Additionally, it should be noted that the memory may be a Double Data Rate (DDR) memory. Due to the large gene data and the limited server memory, it is often extended to the DDR memory. The data is respectively transmitted at a rising edge and a falling edge of clock signal in the DDR memory, leading to that a data transmission speed of the DDR memory is twice that of a conventional SDRAM. Moreover, the energy consumption is not increased, as only the falling edge signal is additionally adopted. The addressing and control signals are identical to those of the conventional SDRAM and are transmitted only on the rising edge of clock signal.

S130: data processing and detection analysis is performed on the slice data by calling an encapsulation program in memory, to obtain an analysis result.

In this step, when processing the slice data, the pre-set encapsulation program in memory needs to be called to the perform data processing and detection analysis on the slice data, and a corresponding analysis result is obtained.

It should be noted that the encapsulation program herein refers to as a software encapsulating a plurality of programs in one program, and the encapsulation program is a custom program, which may encapsulate a plurality of different programs in one program according to user's requirements, for performing the data processing and detection analysis on the slice data.

In addition, the encapsulation program herein is optimized based on the existing internationally recognized program, thereby increasing memory cohesion, increasing multithreading, and adding the processing functions of slice data. During the data processing by using the encapsulation program, the corresponding encapsulation program in a disk can be enabled and loaded into the memory as a task process, thereby effectively reducing I/O consumption, reducing reading and writing time, and improving analysis efficiency.

For example, it is necessary to perform the data processing on the slice data before the slice data is subjected to the detection analysis, and the data processing process can be implemented by one or more programs, such as a data quality control program, a data processing program, etc. Thereafter, a detection analysis program is connected to perform the detection analysis on a result obtained after the data processing. In this way, the final analysis result can be more accurate.

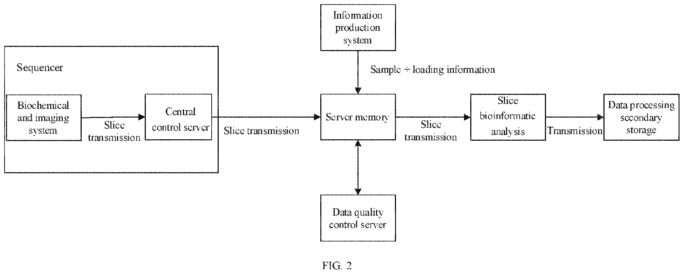

FIG. 2 is a schematic diagram of a flow analysis process according to an embodiment of the present disclosure. As illustrated in FIG. 2,
a sequencer can use the biochemical and imaging system to perform biochemical imaging on the sequencing library, and transmit the images obtained after the biochemical imaging to a central control server in a slice transmission manner. The central control server can transmit the images to an analysis instrument for data processing and detection analysis. The data quality control process includes that, a server memory in a data quality control server obtains the sample and sequencing information in a production information system and the slice data transmitted by the central control server; the data quality control is performed on the slice data, followed by data archiving and delivery; and finally, the analysis result is stored, thereby realizing the flow analysis process.

It can be seen from FIG. 2 that, the set of short sequences transmitted from the sequencing platform is sliced, a part of the analysis time is hided within the original sequencing time, and the data processing process and the detection analysis process are linked to each other via the memory. In this way, the flow analysis process used in the present disclosure can effectively reduce I/O consumption, shorten reading and writing time, and improve analysis efficiency.

With the gene sequencing analysis method provided in the above-mentioned embodiment, short sequences transmitted from a sequencing platform in real time are first sliced to perform corresponding slicing processing, and then the slice data is input into a memory. Thus, a pre-loaded encapsulation program in the memory can be called to perform bioinformatic analysis on the slice data, thereby obtaining a corresponding analysis result. Such a process can acquire the slice data of the sequencing of the sequencing platform in real time and perform sequencing and analysis on the slice data, rather than performing the processing analysis only after the sequencing platform has finished the sequencing and the complete sequencing results are transmitted to the corresponding platform. Therefore, the present disclosure enables the whole of sequencing and analysis to be accelerated. In addition, the data for analysis is the slice data, which can be transmitted faster and takes less time compared with the whole sequencing result.

In addition, the analysis process according to the present disclosure is performed by means of a pre-set encapsulation program in memory. Thus, the sequencing data is unnecessarily transmitted to another platform for processing, which effectively reduces I/O congestion and memory consumption, thereby further improving the operating efficiency of a machine and prolonging the service life of the machine.

The gene sequencing analysis method of the present disclosure will be further explained by means of the following embodiments. As described below, the process of acquiring slice data is described in detail in the following embodiments.

In an embodiment, the step of acquiring slice data in step S110 may include the following steps:
S111: a slice size is determined.

S112: based on the slice size, all unsliced short sequences, which are currently transmitted from the sequencing platform and read after a single cycle of sequencing, are sliced, and the sliced short sequences are used as the slice data.

In this embodiment, the biochemical and imaging parts in the sequencing process of each cycle are transmitted to the memory in real time for base recognition. After the corresponding short sequences are obtained, it is required to determine the slice size first, and the short sequences satisfying the slice size, as a combination, is output as the slice data, and the slice data is stored in the memory.

For example, every m + n cycles (cycles of sequencing) are taken as one piece of slice data, where m is the first slice, m> = 10, n refers to all the subsequent slices, n> = 1 and n <= 20, and m + n can adaptively adjust the slice size according to the analysis speed.

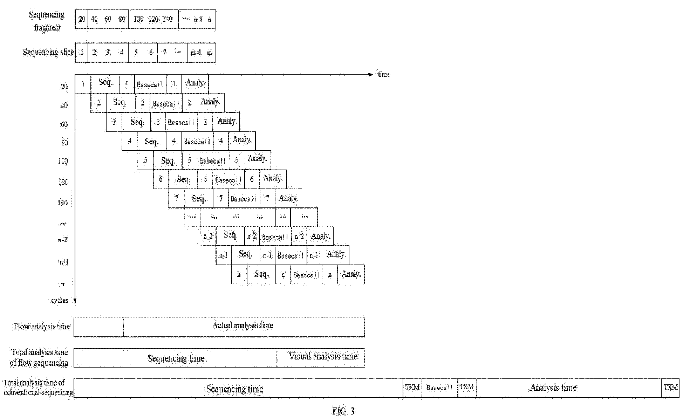

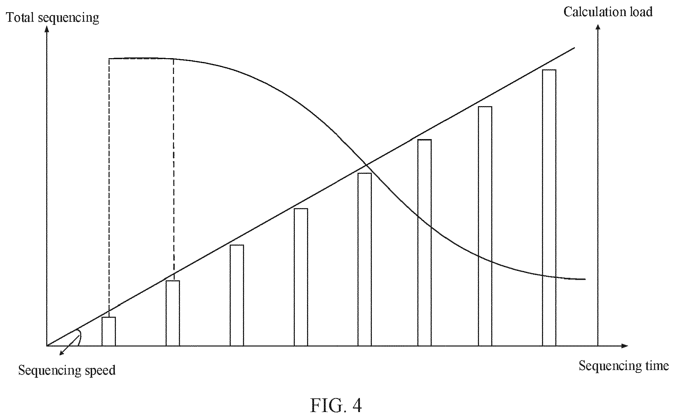

FIG. 3 is a schematic diagram of a gene sequencing analysis process according to an embodiment of the present disclosure, and FIG. 4 is a schematic diagram of a sequencing process in correlation with time in a gene sequencing analysis process according to an embodiment of the present disclosure. As illustrated in FIG. 3 and FIG. 4, in the present disclosure, by acquiring the slice data from the sequencing on a sequencing platform in real time and performing the flow sequencing and analysis on the slice data, the process of sequencing and analysis can be accelerated. Thereafter, a mutation detection or an alignment to an RNA pathogen library, and then subsequent annotation are performed, thereby ending the analysis process, and completing the delivery.

The process of acquiring the slice data is described in detail in the above embodiment. The step of determining the slice size in the above embodiment will be further described below.

In an embodiment, the step of determining the slice size in step S111 may include the following steps.

S1111: a current central processing unit (CPU) idleness state is acquired.

S1112: the slice size is adjusted based on the current CPU idleness state.

In the present embodiment, when the slice data is acquired for detection analysis, the slice data is a set of short sequences transmitted from a sequencing platform in real time and read after performing at least one cycle of sequencing on gene fragments in a sequencing library.

The slice size can be adaptively adjusted according to the analysis speed, and the analysis speed can be obtained by acquiring and analyzing the current CPU idleness state. The current CPU idleness state is related to a current occupation of the memory. More current occupation of the memory indicates that the memory is not idle, and thus the analysis speed of the memory is relatively slow. Therefore, the slice size can be adjusted to be able to slice a set of short sequences that are read after multiple cycles of sequencing. Less current occupation of the memory indicates that the memory is idle, and thus the analysis speed of the memory is relative fast. Therefore, the slice size can be adjusted to be able to slice a set of short sequences that are read after one cycle of sequencing.

For example, every m + n cycles (cycles of sequencing) are taken as one piece of slice data, and the slice after m slices is automatically controlled. That is, a size of the slice n is determined based on the idleness state of the machine from the beginning of the second slice to the end of the last slice. In the machine is relatively idle, the transmission is performed once after each cycle, i.e., n = 1; and if the machine is relatively busy, the transmission is performed after 20 cycle, i.e., n = 20.

It can be understood that, according to the alignment principle in the present disclosure, the slice data is transmitted to the alignment process and aligned with the human genome, and the alignment range of the slice data is determined. If the slice data is larger, the alignment range is reduced more accurately.

The steps of determining the slice size are further explained in the above embodiments, and the encapsulation program and the process of calling the encapsulation program for data processing and detection analysis will be described in detail in the following embodiments.

In an embodiment, the encapsulation program may include a data quality control program, a data processing program, and a detection analysis program.

In step S130, the step of performing, by calling the encapsulation program pre-set in the memory, the data processing and detection analysis on the slice data includes the following steps.

S131: quality control processing is performed on the slice data by calling the data quality control program pre-set in the memory, to obtain a quality control result.

S132: data processing is performed on the quality control result by calling the data processing program pre-set in the memory, to obtain a processing result.

S133: detection analysis is performed on the processing result by calling the detection analysis program pre-set in the memory, to obtain a final analysis result.

In this embodiment, when processing the slice data, the pre-set encapsulation program in memory may be called to perform the data processing and detection analysis on the slice data, to obtain the corresponding analysis result.

The encapsulation program pre-set in the memory may be a custom program. The custom program may be one program encapsulated by multiple programs. For example, the encapsulation program may include a data quality control program, a data processing program, and a detection analysis program.

The data quality control program is mainly used to perform the quality control processing on the slice data, to obtain the quality control result. The data processing program is mainly used to perform the data processing on the quality control result, to obtain the processing result. The detection analysis program is used to perform the detection analysis on the processing result, to obtain the final analysis result.

In the above-mentioned embodiments, the encapsulation program, and the process of performing the data processing and detection analysis by calling the encapsulation program are described in detail. The process of performing the quality control processing by calling the data quality control program is described in detail below.

In an embodiment, the step S131 of performing, by calling the data quality control program pre-set in the memory, the quality control processing on the slice data may include the following steps: by calling the data quality control program pre-set in the memory, calculating a position error rate of short sequence in the slice data, statistically analyzing a base distribution in the slice data, and/or removing low-quality bases from the slice data. The low-quality bases are bases with a base quality below a quality threshold.

In this embodiment, when performing the quality control processing on the slice data, the data quality control program in an encapsulation program is called for performing the quality control processing.

Specifically, the data quality control program is mainly used to perform the quality control processing on the slice data, and the quality control processing may include: calculating the position error rate of the short sequence in the slice data, statistically analyzing the base distribution in the slice data, removing the low-quality bases from the slice data, and other quality control processing applicable to the short sequences. One or more quality control processing and tools to be used in the quality control processing can be specifically selected according to actual conditions.

For example, when the low-quality bases need to be removed from the slice data, the average quality of the bases in a window with a certain length can be calculated by sliding the window. If the average quality is too low, all bases are directly removed.

In the above-mentioned embodiment, the process of performing the quality control processing by calling the data quality control program is described in detail. The process of performing the data processing by calling the data processing program is described in detail by way of embodiments.

In an embodiment, the step S132 of performing, by calling the data processing program pre-set in the memory, the data processing on the quality control result may include: performing, by calling the data processing program pre-set in the memory, at least one of alignment, ordering, deduplication, and re-alignment on the short sequence in the quality control result.

In this embodiment, after the quality control processing is performed on the slice data, the data processing program pre-set in the memory may be called to perform the alignment, ordering, deduplication, and/or re-alignment on the short sequences in the quality control result obtained after the quality control processing.

Although the slice data are all from a genome having a sequence, different short sequences in the slice data has completely lost their sequential relation after DNA library construction and sequencing. Therefore, two adjacent short sequences in the slice data are random short sequences at certain positions in the original genome, and the positional relation between two adjacent short sequences in the slice data is unknown.

Therefore, in the present disclosure, after performing the quality control processing on the slice data, the data processing program pre-set in memory may be called, to align the short sequences in the slice data corresponding to the quality control result with the reference gene of the corresponding species one by one, thereby finding the position of each short sequence on the reference gene. Thereafter, the short sequences are arranged in an order. Such a process is referred to as an alignment process of the slice data.

It should be noted that the reference gene described herein refers to the genomic sequence of the species, i.e., the assembled complete genomic sequence, which is often used as a standard reference for the species.

After the alignment of the short sequences is completed, the short sequences are subjected to a sorting procedure. The sorting procedure is mainly to sort the previously aligned short sequences according to the order of their positions, to form a continuous position relation of the short sequences.

After the ordering procedure, a deduplication operation may be performed on the short sequences in the slice data, in order to remove the repeated short sequences in the slice data, thereby facilitating the subsequent detection analysis.

Finally, the re-alignment process of short sequences is mainly to re-correct the regions of potential sequence insertions or deletions, which have been found in the above-mentioned alignment process, thereby allowing the result of subsequent detection analysis to be more accurate.

It can be understood that the above-mentioned data processing processes of alignment, ordering, deduplication, and re-alignment may be selectively set according to actual situations, and the sequential order may also be manually set, which are not specifically limited herein.

For example, the normal analysis procedures are alignment, ordering, deduplication, re-alignment, mutation detection, and annotation. During the alignment, the input file is a fastq file, and the output file is a bam file. The output is continuously a bam file until the re-alignment. The output file of the mutation detection is a vcf file, and the annotation is based on the result of the mutation detection. When the gene sequencing analysis method of the present disclosure and the next-generation sequencing platform for sequencing are used, the alignment is required for the analysis processes of all raw data of the next-generation fastq, followed by subsequent processes or not. The bam file at any stage can also be input for subsequent analysis processing when not starting from the raw data.

The alignment process of the present disclosure will be further described below. For example, in the alignment of the slice data according to the present disclosure, the bases of the short sequences in the slice data can be aligned with the whole genome to find the position of each short sequence on the reference gene. After all the slice data are positioned at specific positions on the whole genome, the matching alignment information is integrated to perform screening and extension on adjacent alignment positions. In addition, gaps (i.e., the positions where the alignment fails) are allowable, and thus a longer sequence chain aligned to the whole genome can be obtained.

Furthermore, in the above-mentioned alignment process, a dynamic programming algorithm integrating global and local alignment situations can be used to select the optimal alignment result information, and then the alignment result of different samples is respectively output to the memory according to the adapter information.

For example, the common BWA-MEM algorithm mainly uses a seed-and-extend strategy. In a seed stage, BWA takes the base fragment of reads to perform an accurate mapping on reference and selects the read fragment satisfying certain requirements on matching times and length as seed. This stage algorithm focuses on the accurate matching of FM-index. In an extend stage, BWA uses the Smith-Waterman algorithm to extend the seed on reads and reference toward both sides for the alignment (gap-tolerant), thereby finding the eligible global match of the entire read on reference.

The above embodiments mainly describe how the data processing program is called to perform the data processing procedure, and specific embodiments of the data processing will be described further below.

In an embodiment, the step of performing, by calling the data processing program pre-set in the memory, at least one of alignment, ordering, deduplication, and re-alignment on the short sequence in the quality control result may include the following steps.
A11: an alignment between the short sequence in the quality control result and a reference gene is performed by calling the data processing program pre-set in the memory, to determine a position of the short sequence on the reference gene
A12: a position tag of the short sequence is determined based on the position of the short sequence on the reference gene.
A13: among pre-set threads in the memory, a target thread that is currently in an idle state is determined.
A14: at least one of ordering, deduplication, and re-alignment of the short sequence is performed by calling the target thread and with reference to the position tag of the short sequence.

In this embodiment, when performing the data processing on the short sequence in a quality control result, the position tag can be added to the aligned short sequence; and then it is determined whether there is a thread in an idle state among the respective pre-set threads in the current memory. If there is a thread in the idle state, this thread serves as the target thread, and with reference to the position tag of the short sequence, the target thread is called to perform the ordering, deduplication, and/or re-alignment on the short sequence. In this way, the ordering and deduplication are more efficient.

For example, after the short sequence in the quality control result is aligned with the reference gene by calling the data processing program and the position label corresponding to the short sequence is determined based on the position of the short sequence on the reference gene, the target thread can be called to perform operations such as ordering and deduplication on the short sequence. For example, in the ordering process, when multi-thread is used for processing, the ordering can be performed with reference to the position tag of the short sequence, in order to improve the processing efficiency of multi-thread. In this way, the ordering result obtained after multi-thread ordering is accurate and the ordering efficiency is higher.

The process of the data processing is further explained in the above embodiments, and the process of performing the detection analysis on the processing result by calling the detection analysis program is described in detail below.

In an embodiment of the present disclosure, the step S133 of performing, by calling the detection analysis program pre-set in the memory, the detection analysis on the processing result may include: performing, by calling the detection analysis program pre-set in the memory, mutation detection and interpretation on the processing result.

In this embodiment, one goal of performing the gene sequencing analysis is to obtain an accurate mutation set of the sample, which needs to be determined by mutation detection, interpretation, etc. Currently, the results of mutation detection include SNP, Indel, CNV, and SV. The process of mutation detection may use algorithms applying Bayesian inference or HaplotypeCaller algorithm.

For example, when the HaplotypeCaller algorithm is employed for the mutation detection, the haploid combination of the population will be inferred, a probability of each combination is calculated, and then the genotype combination of each sample will be deduced according to this information.

Furthermore, when performing the detection analysis such as the mutation detection on the processing result, the mutation detection can be performed without waiting for the completion of alignment. Different chromosomes in the whole genome can be divided into several regions. When a part of the alignment results is accumulated for each region, the high-mutation region can be found for detection. The continuously verified mutation situation of the existing high-mutation region, including mismatch/insertion/deletion and the like is added into the subsequent alignment result. After an assembly of the data of the high-mutation region, the simplified haplotype data can be obtained. The maximum likelihood estimation of haplotype is estimated by using Hidden Markov Model (HMM) to obtain the typing result of each site in this region, and the mutation information is output.

It can be understood that, in order to improve the analysis efficiency of the whole bioinformatic analysis, when performing the mutation detection on the processing result in the present disclosure, the mutation detection is performed on the high-mutation region rather than on all the sites in the whole genome. The high-mutation region can be selected based on the probability of mutation occurrence at each site. For example, after calculating the probability of mutation occurrence at each site, the high-mutation region can be determined based on a predetermined probability threshold.

Furthermore, during the assembly of the data of the high-mutation region, if there is a duplication in the set of fragments corresponding to the reference genome, the length of the short fragment will be increased progressively until no duplication exits or reaching the maximum length limit, and the edge obtained by the assembly can be assigned with a weight according to the number of short sequences obtained by alignment.

Still further, after obtaining the mutation detection result, the mutation detection result may be subjected to a quality control and filtering, in order to further judge the quality of the mutation detection result.

The gene sequencing analysis apparatus provided in the embodiments of the present disclosure is described below. The gene sequencing analysis apparatus described below can be correspondingly referred to the gene sequencing analysis method described above

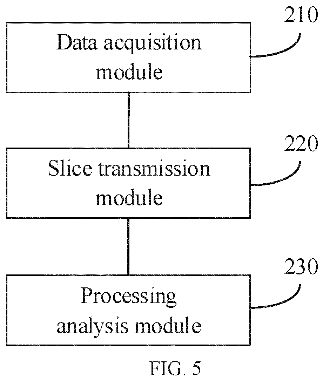

FIG. 5 is a schematic structural diagram of a gene sequencing analysis apparatus according to an embodiment of the present disclosure. In an embodiment, as illustrated in FIG. 5, the present disclosure also provides a gene sequencing analysis apparatus, which includes a data acquisition module 210, a slice transmission module 220, and a processing analysis module 230.

The data acquisition module 210 is configured to acquire slice data transmitted from a sequencing platform in real time, the slice data being a set of short sequences read after performing at least one cycle of sequencing on a sequencing library.

The slice transmission module 220 is configured to input the slice data into a memory.

The processing analysis module 230 is configured to perform, by calling an encapsulation program pre-set in the memory, data processing and detection analysis on the slice data, to obtain a corresponding analysis result, wherein the encapsulation program is a custom program.

With the gene sequencing analysis apparatus provided in the above-mentioned embodiment, short sequences transmitted from a sequencing platform in real time are first sliced to perform corresponding slicing processing, and then the slice data is input into a memory. Thus, a pre-loaded encapsulation program in the memory can be called to perform bioinformatic analysis on the slice data, thereby obtaining a corresponding analysis result. Such a process can acquire the slice data of the sequencing of the sequencing platform in real time and perform sequencing and analysis on the slice data, rather than performing the processing analysis only after the sequencing platform has finished the sequencing and the complete sequencing results are transmitted to the corresponding platform. Therefore, the present disclosure enables the whole of sequencing and analysis to be accelerated. In addition, the data for analysis is the slice data, which can be transmitted faster and takes less time compared with the whole sequencing result.

In addition, the analysis process according to the present disclosure is performed by means of a pre-set encapsulation program in memory. Thus, the sequencing data is unnecessarily transmitted to another platform for processing, which effectively reduces I/O congestion and memory consumption, thereby further improving the operating efficiency of a machine and prolonging the service life of the machine.

In an embodiment, the data acquisition module 210 may include a determination module 211 configured to determine a slice size, and a slicing module 212 configured to slice, based on the slice size, all unsliced short sequences currently transmitted from the sequencing platform and read after a single cycle of sequencing, a set of the sliced short sequences being used as the slice data.

In an embodiment, the determination module 211 may include a memory condition acquisition module 2111 configured to acquire a current idleness state of the memory, and a slice adjustment module 2112 configured to adjust the slice size based on the current idleness state of the memory.

In an embodiment, the encapsulation program in the processing analysis module 230 may include a data quality control program, a data processing program, and a detection analysis program.

The processing analysis module 230, which is configured to perform, by calling an encapsulation program pre-set in the memory, data processing and detection analysis on the slice data, to obtain a corresponding analysis result, may include: a data quality control module 231 configured to perform, by calling the data quality control program pre-set in the memory, quality control processing on the slice data, to obtain a quality control result; a data processing module 232 configured to perform, by calling the data processing program pre-set in the memory, data processing on the quality control result, to obtain a processing result; and a detection analysis module 233 configured to perform, by calling the detection analysis program pre-set in the memory, detection analysis on the processing result, to obtain a final analysis result.

In an embodiment, the data quality control module 231 may include a first processing module 2311. The first processing module 2311 is configured to, by calling the data quality control program pre-set in the memory, calculate a position error rate of short sequence in the slice data, statistically analyze a base distribution in the slice data, and/or remove low-quality bases from the slice data. The low-quality bases are bases with a base quality below a quality threshold.

In an embodiment, the data processing module 232 may include a second processing module 2321. The second processing module 2321 is configured to perform, by calling the data processing program pre-set in the memory, at least one of alignment, ordering, deduplication, and re-alignment on the short sequence in the quality control result.

In an embodiment, the second processing module 2321 may include an alignment module 310, a determination tag module 311, a determining thread module 312, and a multi-thread operation module 313.

The alignment module 310 is configured to perform, by calling the data processing program pre-set in the memory, an alignment between the short sequence in the quality control result and a reference gene, to determine a position of the short sequence on the reference gene.

The determination tag module 311 is configured to determine a position tag of the short sequence based on the position of the short sequence on the reference gene.

The determining thread module 312 is configured to determine, among pre-set threads in the memory, a target thread that is currently in an idle state.

The multi-thread operation module 313 is configured to perform, by calling the target thread and with reference to the position tag of the short sequence, at least one of ordering, deduplication, and re-alignment of the short sequence.

In an embodiment, the detection analysis module 233 may include a third processing module 2331. The third processing module 2331 is configured to perform, by calling the detection analysis program pre-set in the memory, mutation detection and interpretation on the processing result.

In an embodiment, the present disclosure also provides a storage medium having computer readable instructions stored thereon. The computer readable instructions, when being executed by one or more processors, cause the one or more processors to perform steps of the gene sequencing analysis method as described in any of the above embodiments.

In an embodiment, the present disclosure also provides computer device having computer readable instructions stored thereon. The computer readable instructions, when being executed by one or more processors, cause the one or more processors to perform steps of the gene sequencing analysis method as described in any of the above embodiments.

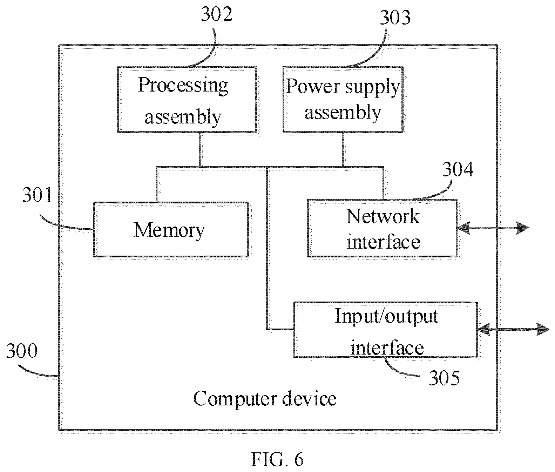

FIG. 6 is a schematic diagram of an internal structure of a computer device according to an embodiment of the present disclosure. The computer device 300 may be provided as a server. Referring to FIG. 6, the computer device 300 includes: a processing component 302, which further includes one or more processors; and a memory resource represented by memory 301 and configured to store an instruction such as an application program, which is executable by the processing component 302. The application program stored in the memory 301 may include one or more modules, each of which corresponds to a set of instructions. Further, the processing component 302 is configured to execute an instruction to perform the gene sequencing analysis method as described in any of the embodiments described above.

The computer device 300 may further include a power supply component 303 configured to perform power management of the computer device 300, a wired or wireless network interface 304 configured to connect the computer device 300 to a network, and an input/output (I/O) interface 305. The computer device 300 may run an operating system stored in memory 301, such as Windows Server TM, Mac OS XTM, Unix TM, Linux TM, Free BSDTM, or the like.

It will be appreciated by those skilled in the art that the structure illustrated in FIG. 6 is merely a block diagram of a part of the structure relevant to the solution of the present disclosure and does not constitute a limitation on the computer device to which the solution of the present disclosure is applied. The specific computer device may include components than those illustrated in the figures, or combine some components, or have a different arrangement of components.

Finally, it should be noted that in this specification, relational terms such as "first" and "second" are used only to differentiate an entity or operation from another entity or operation, and these terms do not necessarily require or imply that any actual relation or sequence exists between these entities or operations. Furthermore, the terms "include", "comprise", or any other mutation thereof, are intended to cover a non-exclusive inclusion, such that a process, method, article, or equipment that includes a list of elements may further include other elements that are not expressly listed or inherent to this process, method, article, or equipment. Without more limitations, the elements defined by the expression "including a..." do not exclude the existence of other identical elements in the process, method, article, or equipment including the elements.

Various embodiments are described in this specification in a progressive manner, with each embodiment focusing on differences from the other embodiments, and these embodiments may be combined as needed, with the same and similar parts referring to each other.

The above embodiments of the present disclosure are provided to enable those skilled in the art to implement or practice the present disclosure.

## Claims

1. A gene sequencing analysis method, comprising:
acquiring slice data transmitted from a sequencing platform in real time, the slice data being a set of short sequences read after performing at least one cycle of sequencing on a sequencing library;
inputting the slice data into a memory; and
performing, by calling an encapsulation program pre-set in the memory, data processing and detection analysis on the slice data, to obtain a corresponding analysis result, wherein the encapsulation program is a custom program;
wherein the encapsulation program comprises a data quality control program, a data processing program, and a detection analysis program, and
wherein said performing, by calling the encapsulation program pre-set in the memory, the data processing and detection analysis on the slice data, to obtain the corresponding analysis result comprises:
performing, by calling the data quality control program pre-set in the memory, quality control processing on the slice data, to obtain a quality control result;
performing, by calling the data processing program pre-set in the memory, data processing on the quality control result, to obtain a processing result; and
performing, by calling the detection analysis program pre-set in the memory, detection analysis on the processing result, to obtain a final analysis result;
wherein said acquiring the slice data comprises:
determining a slice size;
slicing, based on the slice size, all unsliced short sequences currently transmitted from the sequencing platform and read after a single cycle of sequencing, a set of the sliced short sequences being used as the slice data;
wherein said determining the slice size comprises:
acquiring a current central processing unit (CPU) idleness state; and
adjusting the slice size based on the current CPU idleness state;
wherein when the memory is not idle, the slice size is adjusted to be able to slice a set of short sequences that are read after multiple cycles of sequencing; and when the memory is idle, the slice size is adjusted to be able to slice a set of short sequences that are read after one cycle of sequencing;
wherein biochemical and imaging parts in sequencing process of each cycle are transmitted to the memory in real time for base recognition, after corresponding short sequences are obtained, the slice size is determined first, and the short sequences satisfying the slice size, as a combination, is output as the slice data, and the slice data is stored in the memory;
wherein every set of multiple cycles of sequencing is taken as one piece of slice data, divided into a first slice and subsequent slices, and the slice size can be adaptively adjusted according to the analysis speed; where the first slice contains multiple sequencing cycles; the subsequent slices are automatically controlled: if the machine is relatively idle, the transmission is performed once after each cycle, the subsequent slices only contain one sequencing cycle; and if the machine is relatively busy, the transmission can be selected to perform after a suitable sequencing cycle number of the subsequent slices.

2. The gene sequencing analysis method according to claim 1, wherein said performing, by calling the data quality control program pre-set in the memory, the quality control processing on the slice data comprises:
by calling the data quality control program pre-set in the memory, calculating a position error rate of short sequences in the slice data, statistically analyzing a base distribution in the slice data, and/or removing low-quality bases from the slice data, wherein the low-quality bases are bases with a base quality below a quality threshold.

3. The gene sequencing analysis method according to claim 1, wherein said performing, by calling the data processing program pre-set in the memory, the data processing on the quality control result comprises:
performing, by calling the data processing program pre-set in the memory, at least one of alignment, ordering, deduplication, re-alignment, and base quality value correction on a short sequence in the quality control result.

4. The gene sequencing analysis method according to claim 3, wherein said performing, by calling the data processing program pre-set in the memory, at least one of alignment, ordering, deduplication, and re-alignment on the short sequence in the quality control result comprises:
performing, by calling the data processing program pre-set in the memory, an alignment between the short sequence in the quality control result and a reference gene, to determine a position of the short sequence on the reference gene;
determining a position tag of the short sequence based on the position of the short sequence on the reference gene;
determining, among pre-set threads in the memory, a target thread that is currently in an idle state; and
performing, by calling the target thread and with reference to the position tag of the short sequence, at least one of ordering, deduplication, and re-alignment of the short sequence.

5. The gene sequencing analysis method according to claim 1, wherein said performing, by calling the detection analysis program pre-set in the memory, the detection analysis on the processing result comprises:
performing, by calling the detection analysis program pre-set in the memory, mutation detection and interpretation on the processing result.

6. A gene sequencing analysis apparatus, comprising:
a data acquisition module configured to acquire slice data transmitted from a sequencing platform in real time, the slice data being a set of short sequences read after performing at least one cycle of sequencing on a sequencing library;
a slice transmission module configured to input the slice data into a memory; and
a processing analysis module configured to perform, by calling an encapsulation program pre-set in the memory, data processing and detection analysis on the slice data, to obtain a corresponding analysis result, wherein the encapsulation program is a custom program;
wherein the encapsulation program comprises a data quality control program, a data processing program, and a detection analysis program, and
wherein the processing analysis module is further configured to:
perform, by calling the data quality control program pre-set in the memory, quality control processing on the slice data, to obtain a quality control result;
perform, by calling the data processing program pre-set in the memory, data processing on the quality control result, to obtain a processing result; and
perform, by calling the detection analysis program pre-set in the memory, detection analysis on the processing result, to obtain a final analysis result;
wherein the data acquisition module is further configured to:
determine a slice size;
slice, based on the slice size, all unsliced short sequences currently transmitted from the sequencing platform and read after a single cycle of sequencing, a set of the sliced short sequences being used as the slice data;
wherein for determining the slice size, the data acquisition module is further configured to:
acquire a current central processing unit (CPU) idleness state; and
adjust the slice size based on the current CPU idleness state;
wherein when the memory is not idle, the slice size is adjusted to be able to slice a set of short sequences that are read after multiple cycles of sequencing; and when the memory is idle, the slice size is adjusted to be able to slice a set of short sequences that are read after one cycle of sequencing;
wherein biochemical and imaging parts in sequencing process of each cycle are transmitted to the memory in real time for base recognition, after corresponding short sequences are obtained, the slice size is determined first, and the short sequences satisfying the slice size, as a combination, is output as the slice data, and the slice data is stored in the memory;
wherein every set of multiple cycles of sequencing is taken as one piece of slice data, divided into a first slice and subsequent slices, and the slice size can be adaptively adjusted according to the analysis speed; where the first slice contains multiple sequencing cycles; the subsequent slices are automatically controlled: if the machine is relatively idle, the transmission is performed once after each cycle, the subsequent slices only contain one sequencing cycle; and if the machine is relatively busy, the transmission can be selected to perform after a suitable sequencing cycle number of the subsequent slices.

7. A storage medium, having computer readable instructions stored thereon, wherein the computer readable instructions, when being executed by one or more processors, cause the one or more processors to perform steps of the gene sequencing analysis method according to any one of claims 1 to 5.

8. A computer device, having computer readable instructions stored thereon, wherein the computer readable instructions, when being executed by one or more processors, cause the one or more processors to perform steps of the gene sequencing analysis method according to any one of claims 1 to 5.

## Patentansprüche

1. Verfahren zur Analyse von Gensequenzierungen, umfassend:
Erfassen von Slice-Daten, die in Echtzeit von einer Sequenzierungsplattform übertragen werden, wobei die Slice-Daten eine Menge kurzer Sequenzen sind, die nach Durchführung mindestens eines Sequenzierungszyklus einer Sequenzierungsbibliothek gelesen wurden;
Einspeisen der Slice-Daten in einen Speicher; und
Durchführen, durch Aufruf eines im Speicher voreingestellten Kapselungsprogramms, einer Datenverarbeitung und Detektionsanalyse der Slice-Daten, um ein entsprechendes Analyseergebnis zu erhalten, wobei das Kapselungsprogramm ein benutzerdefiniertes Programm ist;
wobei das Kapselungsprogramm ein Datenqualitätskontrollprogramm, ein Datenverarbeitungsprogramm und ein Detektionsanalyseprogramm umfasst, und
wobei das Durchführen, durch Aufruf des im Speicher voreingestellten Kapselungsprogramms, der Datenverarbeitung und Detektionsanalyse der Slice-Daten zum Erhalt des entsprechenden Analyseergebnisses Folgendes umfasst:
Durchführen, durch Aufruf des im Speicher voreingestellten Datenqualitätskontrollprogramms, einer Qualitätskontrollverarbeitung der Slice-Daten, um ein Qualitätskontrollergebnis zu erhalten;
Durchführen, durch Aufruf des im Speicher voreingestellten Datenverarbeitungsprogramms, einer Datenverarbeitung des Qualitätskontrollergebnisses, um ein Verarbeitungsergebnis zu erhalten; und
Durchführen, durch Aufruf des im Speicher voreingestellten Detektionsanalyseprogramms, einer Detektionsanalyse des Verarbeitungsergebnisses, um ein finales Analyseergebnis zu erhalten;
wobei das Erfassen der Slice-Daten Folgendes umfasst:
Bestimmen einer Slice-Größe;
Schneiden, basierend auf der Slice-Größe, aller derzeit von der Sequenzierungsplattform übertragenen und nach einem einzelnen Sequenzierungszyklus gelesenen, ungeschnittenen kurzen Sequenzen, wobei ein Satz der geschnittenen kurzen Sequenzen als Slice-Daten verwendet wird;
wobei das Bestimmen der Slice-Größe Folgendes umfasst:
Erfassen eines aktuellen Leerlaufzustands einer zentralen Verarbeitungseinheit (CPU); und
Anpassen der Slice-Größe basierend auf dem aktuellen Leerlaufzustand der CPU;
wobei, wenn der Speicher nicht im Leerlauf ist, die Slice-Größe so angepasst wird, dass ein Satz kurzer Sequenzen, die nach mehreren Sequenzierungszyklen gelesen wurden, geschnitten werden kann; und wenn der Speicher im Leerlauf ist, die Slice-Größe so angepasst wird, dass ein Satz kurzer Sequenzen, die nach einem Zyklus gelesen wurden, geschnitten werden kann;
wobei biochemische und bildgebende Teile des Sequenzierungsprozesses jedes Zyklus in Echtzeit zur Basenerkennung an den Speicher übertragen werden, nachdem entsprechende kurze Sequenzen erhalten wurden, wird zuerst die Slice-Größe bestimmt, und die kurzen Sequenzen, die der Slice-Größe entsprechen, werden als Kombination als Slice-Daten ausgegeben, und die Slice-Daten werden im Speicher gespeichert;
wobei jeder Satz aus mehreren Sequenzierungszyklen als ein Satz von Slice-Daten genommen wird, aufgeteilt in einen ersten Slice und nachfolgende Slices, und die Slice-Größe adaptiv entsprechend der Analysegeschwindigkeit angepasst werden kann; wobei der erste Slice mehrere Sequenzierungszyklen enthält; die nachfolgenden Slices automatisch gesteuert werden: wenn die Maschine relativ im Leerlauf ist, erfolgt die Übertragung einmal nach jedem Zyklus, die nachfolgenden Slices enthalten nur einen Sequenzierungszyklus; und wenn die Maschine relativ ausgelastet ist, kann die Übertragung nach einer geeigneten Anzahl von Sequenzierungszyklen der nachfolgenden Slices ausgewählt werden.

2. Verfahren zur Analyse von Gensequenzierungen nach Anspruch 1, wobei das Durchführen, durch Aufruf des im Speicher voreingestellten Datenqualitätskontrollprogramms, der Qualitätskontrollverarbeitung der Slice-Daten Folgendes umfasst:
durch Aufruf des im Speicher voreingestellten Datenqualitätskontrollprogramms wird eine Positionsfehlerrate der kurzen Sequenzen in den Slice-Daten berechnet, eine Basenverteilung in den Slice-Daten statistisch analysiert und/oder Basen niedriger Qualität aus den Slice-Daten entfernt, wobei die niedrigen Basen niedriger Qualität Basen mit einer Basenqualität unterhalb eines Qualitätsschwellenwerts sind.

3. Verfahren zur Analyse von Gensequenzierungen nach Anspruch 1, wobei das Durchführen, durch Aufruf des im Speicher voreingestellten Datenverarbeitungsprogramms, der Datenverarbeitung des Qualitätskontrollergebnisses Folgendes umfasst:
Durchführen, durch Aufruf des im Speicher voreingestellten Datenverarbeitungsprogramms, von mindestens einem der folgenden Schritte: Ausrichtung, Sortierung, Duplikatentfernung, erneute Ausrichtung und Korrektur des Basenqualitätswerts an einer kurzen Sequenz im Qualitätskontrollergebnis.

4. Verfahren zur Analyse von Gensequenzierungen nach Anspruch 3, wobei das Durchführen, durch Aufruf des im Speicher voreingestellten Datenverarbeitungsprogramms, von mindestens einem der Schritte Ausrichtung, Sortierung, Duplikatentfernung und erneute Ausrichtung an der kurzen Sequenz im Qualitätskontrollergebnis Folgendes umfasst:
Durchführen, durch Aufruf des im Speicher voreingestellten Datenverarbeitungsprogramms, einer Ausrichtung zwischen der kurzen Sequenz im Qualitätskontrollergebnis und einem Referenzgen, um eine Position der kurzen Sequenz auf dem Referenzgen zu bestimmen;
Bestimmen eines Positionstags der kurzen Sequenz basierend auf der Position der kurzen Sequenz auf dem Referenzgen;
Bestimmen eines Ziel-Threads, der sich aktuell in einem Leerlaufzustand befindet, unter voreingestellten Threads im Speicher; und
Durchführen, durch Aufruf des Ziel-Threads und unter Bezugnahme auf den Positionstag der kurzen Sequenz, von mindestens einem der folgenden Schritte: Sortierung, Duplikatentfernung und erneute Ausrichtung der kurzen Sequenz.

5. Verfahren zur Analyse von Gensequenzierungen nach Anspruch 1, wobei das Durchführen, durch Aufruf des im Speicher voreingestellten Detektionsanalyseprogramms, der Detektionsanalyse des Verarbeitungsergebnisses Folgendes umfasst:
Durchführen, durch Aufruf des im Speicher voreingestellten Detektionsanalyseprogramms, von Mutationsdetektion und Interpretation des Verarbeitungsergebnisses.

6. Vorrichtung zur Analyse von Gensequenzierungen, umfassend:
ein Datenerfassungsmodul, das konfiguriert ist, Slice-Daten, die eine Menge kurzer Sequenzen sind, welche nach Durchführung mindestens eines Sequenzierungszyklus auf einer Sequenzierungsbibliothek gelesen wurden, in Echtzeit von einer Sequenzierungsplattform zu erfassen;
ein Slice-Übertragungsmodul, das konfiguriert ist, die Slice-Daten in einen Speicher einzuspeisen; und
ein Verarbeitungsanalysemodul, das konfiguriert ist, durch Aufruf eines im Speicher voreingestellten Kapselungsprogramms, Datenverarbeitung und Detektionsanalyse der Slice-Daten durchzuführen, um ein entsprechendes Analyseergebnis zu erhalten, wobei das Kapselungsprogramm ein benutzerdefiniertes Programm ist;
wobei das Kapselungsprogramm ein Datenqualitätskontrollprogramm, ein Datenverarbeitungsprogramm und ein Detektionsanalyseprogramm umfasst, und wobei das Verarbeitungsanalysemodul ferner konfiguriert ist:
eine Qualitätskontrollverarbeitung der Slice-Daten durch Aufruf des im Speicher voreingestellten Datenqualitätskontrollprogramms durchzuführen, um ein Qualitätskontrollergebnis zu erhalten;
eine Datenverarbeitung des Qualitätskontrollergebnisses durch Aufruf des im Speicher voreingestellten Datenverarbeitungsprogramms durchzuführen, um ein Verarbeitungsergebnis zu erhalten; und
eine Detektionsanalyse des Verarbeitungsergebnisses durch Aufruf des im Speicher voreingestellten Detektionsanalyseprogramms durchzuführen, um ein finales Analyseergebnis zu erhalten;
wobei das Datenerfassungsmodul ferner konfiguriert ist:
eine Slice-Größe zu bestimmen;
basierend auf der Slice-Größe, alle derzeit von der Sequenzierungsplattform übertragenen und nach einem einzelnen Sequenzierungszyklus gelesenen ungeschnittenen kurzen Sequenzen zu schneiden, wobei ein Satz der geschnittenen kurzen Sequenzen als Slice-Daten verwendet wird;
wobei zur Bestimmung der Slice-Größe das Datenerfassungsmodul ferner konfiguriert ist:
einen aktuellen Leerlaufzustand der zentralen Verarbeitungseinheit (CPU) zu erfassen; und
die Slice-Größe basierend auf dem aktuellen Leerlaufzustand der CPU anzupassen;
wobei, wenn der Speicher nicht im Leerlauf ist, die Slice-Größe so angepasst wird, dass ein Satz kurzer Sequenzen, die nach mehreren Sequenzierungszyklen gelesen wurden, geschnitten werden kann; und wenn der Speicher im Leerlauf ist, die Slice-Größe so angepasst wird, dass ein Satz kurzer Sequenzen, die nach einem Sequenzierungszyklus gelesen wurden, geschnitten werden kann;
wobei biochemische und bildgebende Teile des Sequenzierungsprozesses jedes Zyklus in Echtzeit zur Basenerkennung an den Speicher übertragen werden, nachdem entsprechende kurze Sequenzen erhalten wurden, wird zuerst die Slice-Größe bestimmt, und die kurzen Sequenzen, die der Slice-Größe entsprechen, werden kombiniert, als Slice-Daten ausgegeben und im Speicher gespeichert;
wobei jeder Satz aus mehreren Sequenzierungszyklen als ein Satz von Slice-Daten betrachtet wird, unterteilt in einen ersten Slice und nachfolgende Slices, und die Slice-Größe adaptiv entsprechend der Analysegeschwindigkeit angepasst werden kann; wobei der erste Slice mehrere Sequenzierungszyklen enthält; die nachfolgenden Slices automatisch gesteuert werden: wenn die Maschine relativ im Leerlauf ist, erfolgt die Übertragung einmal nach jedem Zyklus, die nachfolgenden Slices enthalten jeweils nur einen Sequenzierungszyklus; und wenn die Maschine relativ ausgelastet ist, kann die Übertragung nach einer geeigneten Anzahl von Sequenzierungszyklen der nachfolgenden Slices erfolgen.

7. Speichermedium mit darauf gespeicherten computerlesbaren Anweisungen, wobei die computerlesbaren Anweisungen, wenn sie von einem oder mehreren Prozessoren ausgeführt werden, bewirken, dass der oder die Prozessoren die Schritte des Gensequenzierungsanalyseverfahrens gemäß einem der Ansprüche 1 bis 5 ausführen.

8. Recheneinheit mit darauf gespeicherten computerlesbaren Anweisungen, wobei die computerlesbaren Anweisungen, wenn sie von einem oder mehreren Prozessoren ausgeführt werden, bewirken, dass der oder die Prozessoren die Schritte des Gensequenzierungsanalyseverfahrens gemäß einem der Ansprüche 1 bis 5 ausführen.

## Revendications

1. Un procédé d'analyse de séquençage génétique, comprenant :
acquérir des données de tranche transmises à partir d'une plateforme de séquençage en temps réel, les données de tranche étant un ensemble de courtes séquences lues après avoir effectué au moins un cycle de séquençage sur une bibliothèque de séquençage ;
entrer les données de tranche dans une mémoire ; et
effectuer, par appel à un programme encapsulé prédéfini dans la mémoire, un traitement de données et une analyse de détection sur les données de tranche, pour obtenir un résultat d'analyse correspondant, ledit programme encapsulé étant un programme personnalisé ;
le programme encapsulé comprenant un programme de contrôle qualité des données, un programme de traitement de données, et un programme d'analyse de détection, et
le fait d'effectuer, par appel au programme encapsulé prédéfini dans la mémoire, le traitement de données et l'analyse de détection sur les données de tranche, pour obtenir le résultat d'analyse correspondant, comprend :
effectuer, par appel au programme de contrôle qualité prédéfini dans la mémoire, un traitement de contrôle qualité sur les données de tranche, pour obtenir un résultat de contrôle qualité ;
effectuer, par appel au programme de traitement de données prédéfini dans la mémoire, un traitement de données sur le résultat de contrôle qualité, pour obtenir un résultat de traitement ; et
effectuer, par appel au programme d'analyse de détection prédéfini dans la mémoire, une analyse de détection sur le résultat de traitement, pour obtenir un résultat final d'analyse ;
le fait d'acquérir les données de tranche comprend :
déterminer une taille de tranche ;
découper, sur la base de la taille de tranche, toutes les courtes séquences non découpées actuellement transmises à partir de la plateforme de séquençage et lues après un seul cycle de séquençage, un ensemble des courtes séquences découpées étant utilisé comme données de tranche ;
le fait de déterminer la taille de tranche comprend :
acquérir un état d'inactivité actuel du processeur central (CPU) ; et
ajuster la taille de tranche en fonction de l'état d'inactivité actuel du CPU ;
lorsque la mémoire n'est pas inactive, la taille de tranche est ajustée pour pouvoir découper un ensemble de courtes séquences lues après plusieurs cycles de séquençage ; et lorsque la mémoire est inactive, la taille de tranche est ajustée pour pouvoir découper un ensemble de courtes séquences lues après un seul cycle de séquençage ;
les parties biochimiques et d'imagerie dans le processus de séquençage de chaque cycle sont transmises à la mémoire en temps réel pour la reconnaissance des bases, après obtention des courtes séquences correspondantes, la taille de tranche est d'abord déterminée, et les courtes séquences satisfaisant la taille de tranche, comme une combinaison, sont sorties comme données de tranche, et les données de tranche sont stockées dans la mémoire ;
chaque ensemble de plusieurs cycles de séquençage est considéré comme une pièce de données de tranche, divisée en une première tranche et des tranches suivantes, et la taille de tranche peut être ajustée de manière adaptative selon la vitesse d'analyse ; la première tranche contenant plusieurs cycles de séquençage ; les tranches suivantes sont automatiquement contrôlées : si la machine est relativement inactive, la transmission est effectuée une fois après chaque cycle, les tranches suivantes ne contenant qu'un seul cycle de séquençage ; et si la machine est relativement occupée, la transmission peut être effectuée après un nombre approprié de cycles de séquençage des tranches suivantes.

2. Le procédé d'analyse de séquençage génétique selon la revendication 1, dans lequel le fait d'effectuer, par appel au programme de contrôle qualité prédéfini dans la mémoire, le traitement de contrôle qualité sur les données de tranche comprend :
par appel au programme de contrôle qualité prédéfini dans la mémoire, calculer un taux d'erreur de position des courtes séquences dans les données de tranche, analyser statistiquement une distribution des bases dans les données de tranche, et/ou supprimer les bases de faible qualité des données de tranche, les bases de faible qualité étant des bases ayant une qualité de base inférieure à un seuil de qualité.

3. Le procédé d'analyse de séquençage génétique selon la revendication 1, dans lequel le fait d'effectuer, par appel au programme de traitement de données prédéfini dans la mémoire, le traitement de données sur le résultat de contrôle qualité comprend :
effectuer, par appel au programme de traitement de données prédéfini dans la mémoire, au moins l'un d'alignement, tri, déduplication, réalignement, et correction de la valeur de qualité de base sur une courte séquence dans le résultat de contrôle qualité.

4. Le procédé d'analyse de séquençage génétique selon la revendication 3, dans lequel le fait d'effectuer, par appel au programme de traitement de données prédéfini dans la mémoire, au moins l'un d'alignement, tri, déduplication, et réalignement sur la courte séquence dans le résultat de contrôle qualité comprend :
effectuer, par appel au programme de traitement de données prédéfini dans la mémoire, un alignement entre la courte séquence dans le résultat de contrôle qualité et un gène de référence, pour déterminer une position de la courte séquence sur le gène de référence ;
déterminer une étiquette de position de la courte séquence sur la base de la position de la courte séquence sur le gène de référence ;
déterminer, parmi les threads prédéfinis dans la mémoire, un thread cible actuellement dans un état d'inactivité ; et
effectuer, par appel au thread cible et en se référant à l'étiquette de position de la courte séquence, au moins l'un de tri, déduplication, et réalignement de la courte séquence.

5. Le procédé d'analyse de séquençage génétique selon la revendication 1, dans lequel le fait d'effectuer, par appel au programme d'analyse de détection prédéfini dans la mémoire, l'analyse de détection sur le résultat de traitement comprend :
effectuer, par appel au programme d'analyse de détection prédéfini dans la mémoire, une détection de mutation et une interprétation sur le résultat de traitement.

6. Un appareil d'analyse de séquençage génétique, comprenant :
un module d'acquisition de données configuré pour acquérir des données de tranche transmises à partir d'une plateforme de séquençage en temps réel, les données de tranche étant un ensemble de courtes séquences lues après avoir effectué au moins un cycle de séquençage sur une bibliothèque de séquençage ;
un module de transmission de tranche configuré pour entrer les données de tranche dans une mémoire ; et
un module d'analyse de traitement configuré pour effectuer, par appel à un programme encapsulé prédéfini dans la mémoire, un traitement de données et une analyse de détection sur les données de tranche, pour obtenir un résultat d'analyse correspondant, ledit programme encapsulé étant un programme personnalisé ;
le programme encapsulé comprenant un programme de contrôle qualité des données, un programme de traitement de données, et un programme d'analyse de détection, et
le module d'analyse de traitement étant en outre configuré pour :
effectuer, par appel au programme de contrôle qualité prédéfini dans la mémoire, un traitement de contrôle qualité sur les données de tranche, pour obtenir un résultat de contrôle qualité ;
effectuer, par appel au programme de traitement de données prédéfini dans la mémoire, un traitement de données sur le résultat de contrôle qualité, pour obtenir un résultat de traitement ; et
effectuer, par appel au programme d'analyse de détection prédéfini dans la mémoire, une analyse de détection sur le résultat de traitement, pour obtenir un résultat final d'analyse ;
le module d'acquisition de données étant en outre configuré pour :
déterminer une taille de tranche ;
découper, sur la base de la taille de tranche, toutes les courtes séquences non découpées actuellement transmises à partir de la plateforme de séquençage et lues après un seul cycle de séquençage, un ensemble des courtes séquences découpées étant utilisé comme données de tranche ;
pour déterminer la taille de tranche, le module d'acquisition de données étant en outre configuré pour :
acquérir un état d'inactivité actuel du processeur central (CPU) ; et
ajuster la taille de tranche en fonction de l'état d'inactivité actuel du CPU ;
lorsque la mémoire n'est pas inactive, la taille de tranche est ajustée pour pouvoir découper un ensemble de courtes séquences lues après plusieurs cycles de séquençage ; et lorsque la mémoire est inactive, la taille de tranche est ajustée pour pouvoir découper un ensemble de courtes séquences lues après un seul cycle de séquençage ;
les parties biochimiques et d'imagerie dans le processus de séquençage de chaque cycle sont transmises à la mémoire en temps réel pour la reconnaissance des bases, après obtention des courtes séquences correspondantes, la taille de tranche est d'abord déterminée, et les courtes séquences satisfaisant la taille de tranche, comme une combinaison, sont sorties comme données de tranche, et les données de tranche sont stockées dans la mémoire ;
chaque ensemble de plusieurs cycles de séquençage est considéré comme une pièce de données de tranche, divisée en une première tranche et des tranches suivantes, et la taille de tranche peut être ajustée de manière adaptative selon la vitesse d'analyse ; la première tranche contenant plusieurs cycles de séquençage ; les tranches suivantes sont automatiquement contrôlées : si la machine est relativement inactive, la transmission est effectuée une fois après chaque cycle, les tranches suivantes ne contenant qu'un seul cycle de séquençage ; et si la machine est relativement occupée, la transmission peut être effectuée après un nombre approprié de cycles de séquençage des tranches suivantes.

7. Un support de stockage, sur lequel sont stockées des instructions lisibles par ordinateur, lesdites instructions, lorsqu'elles sont exécutées par un ou plusieurs processeurs, amenant le ou les processeurs à effectuer les étapes du procédé d'analyse de séquençage génétique selon l'une quelconque des revendications 1 à 5.

8. Un dispositif informatique, sur lequel sont stockées des instructions lisibles par ordinateur, lesdites instructions, lorsqu'elles sont exécutées par un ou plusieurs processeurs, amenant le ou les processeurs à effectuer les étapes du procédé d'analyse de séquençage génétique selon l'une quelconque des revendications 1 à 5.
